# EUROPEAN PATENT SPECIFICATION

(11) **EP 4 609 722 B1**
(45) Date of publication and mention of the grant of the patent: **04.11.2026**
(21) Application number: 25155590.0
(22) Date of filing: 03.02.2025
(51) Int. Cl.: A23K 10/24, A23K 20/163, A23K 50/40, A61P 1/00, A61P 37/04

(54) **ANIMAL FOOD COMPOSITIONS CONTAINING A COMBINATION OF SPRAY-DRIED PLASMA AND FRUCTO-OLIGOSACCHARIDES**
TIERFUTTERZUSAMMENSETZUNGEN MIT EINER KOMBINATION AUS SPRÜHGETROCKNETEM PLASMA UND FRUCTO-OLIGOSACCHARIDEN
COMPOSITIONS ALIMENTAIRES POUR ANIMAUX CONTENANT UNE COMBINAISON DE PLASMA SÉCHÉ PAR ATOMISATION ET DE FRUCTO-OLIGOSACCHARIDES

(30) Priority: 28.02.2024 WO PCT/IB2024/051926
(43) Date of publication of application: 03.09.2025
(73) Proprietor: HHNW Singapore Pte. Limited, 048619 Singapore (SG)
(72) Inventor: LANE, Jonathan, Fermoy, P61K202 (IE); KONDRASHINA, Alina, Fermoy, P61K202 (IE); CASTANEDA GUTIERREZ, Euridice, 1202 Geneva (CH); WALSH, Clodagh, Fermoy, P61K202 (IE); O' HEA, Reuben, Fermoy, P61K202 (IE); BEDI, Akash, P61K202 Fermoy, Co. Cork (IE)
(74) Representative: Straus, Alexander

(56) References cited:
- WO-A1-2016/030861
- CN-A- 108 323 641
- CN-A- 108 902 499
- CN-A- 117 243 298
- DE LANGE C F M ET AL: "Strategic use of feed ingredients and feed additives to stimulate gut health and development in young pigs", LIVESTOCK SCIENCE, ELSEVIER, AMSTERDAM, NL, vol. 134, no. 1-3, 1 September 2010 (2010-09-01), pages 124 - 134, XP027353425, ISSN: 1871-1413, [retrieved on 20100807]

## Description

### BACKGROUND OF THE INVENTION

### Technical Field

The present invention relates to animal food, in particular pet food comprising a combination of spray-dried plasma and fructo-oligosaccharides. The invention further relates to the use of a spray-dried plasma and fructo-oligosaccharides combination containing animal food for improving gastro-intestinal digestion, gut microbial diversity and fermentation, stool consistency and odour, nutrient absorption, and/or immunity in an animal.

### State of the Art

Food is primarily devised to provide the energy required to maintain an individual's physical integrity. Meanwhile it is well acknowledged that apart from its nutritional value the type and nature of food has an impact on an individual's well-being and health condition due to its influence on the gut microbiome.

Animal food, especially pet food mainly consists of corn with meat, bone, soya bean meal and many other ingredients being added to complement the same so as to meet an animal's particular requirements based on a given life stage. Among the elemental nutrients to be added are proteins, essential amino acids, fatty acids, vitamins, minerals, etc.

For providing proteins in animal food, plasma, a fraction of animal blood obtained as a side-product in cattle, lamb, pork or poultry processing for human consumption, has caught a great deal of interest. It contains high concentrations of a variety of proteins, essential amino acids, bioactive peptides growth factors, enzymes, metalloproteins etc. CN117243298 A discloses pet feed capable of improving immunity, comprising a feed basis, a basic immunizing agent and an immunopotentiator, the raw materials of the immunopotentiator comprise pig plasma protein powder, bovine colostrum powder and pepsin, and when the immunopotentiator is used in dog food, the immunopotentiator further comprises wall-broken bee pollen. Said feed can improve the level of immune complements C3 and C4 of the adult cats and dogs through synergistic interaction.

Plasma has a number of advantages in the production of animal food. It may easily be processed by spray-drying without losing its inherent solubility and functional properties so that shipping and inclusion thereof into food is strongly facilitated. It may also serve as an emulsifying and binding agent in the production of wet foods, e.g. pates and chunks in gravy, and may in this way replace hydrocolloids, which have been found to exhibit some negative implications for digestibility, faecal quality, and intestinal inflammation.

Inclusion of SDP in animal food has been associated with positive effects against intestinal pathogens, which effects seem to be mainly related to the activity of immunoglobulins and/or bioactive peptides present therein.

Yet, the most important implication of spray-dried plasma (SDP) in animal food resides in its nutritional value based on its high protein content, and its concerted composition of essential amino acids. Compared with other protein sources, SDP has the highest total amino acid score for lysine, tryptophan, and threonine, which, together with methionine, are the main limiting amino acids in diets for dogs and cats.

In providing animal food with better nutritional value and quality producers conventionally strive on the one hand to increase the amount of proteins in the food, which is yet to some extent accompanied by an increased faecal malodour, especially inconvenient for dog owners living with their animals in-house. The malodour produced is mainly based on volatile substances generated by bacterial degradation of ingested proteins, i.e. ammonia, aliphatic amines, branched chain fatty acids, indoles, phenols, and sulphur-containing compounds.

On the other hand there is a tendency to include further functional compounds in animal food, such as prebiotics, i.e. compounds that pass the upper part of the gut in an essentially undigested form, but may be fermented by micro-organisms present in the colon. As such they serve as substrates and nutrients for these micro-organisms and foster growth and activity thereof, so that other potentially harmful bacteria experience a competitive disadvantage for their growth, with an overall beneficial effect for the individual' health and well-being. The most commonly used prebiotics include polysaccharides, such as beta-glycan, resistant starch and inulin. Yet, due to fermentation processes in the gut microbiota, administration of these substances also tend to increase flatulence, based on the nature thereof and amount ingested. In view of the above there is still a need for an animal food that overcomes the disadvantages of the prior art, and supports the immune system and gut health.

### SUMMARY OF THE INVENTION

The present invention provides an animal food composition comprising Spray-Dried Plasma (SDP) and fructo-oligosaccharides (FOS) in a ratio of from 10 : 2 to 10 : 4. The ratio of SDP to FOS may preferably be in a range of from 10 : 2.5 to 10 : 3.5, most preferably 10 : 3.

The animal food composition may contain the SDP depending on the nature thereof in an amount of from 0.5 % to 90 %, based on the total weight of the dry or wet food product. Likewise the amount of FOS in the animal food compositions may range of from 0.1 % and up to 45 %.

The animal food is preferably formulated as a pet food, specifically formulated as cat or dog food, or as a supplement, a topper, or treat products etc.

The present food composition may be used for treating and/or preventing a gut disorder, such as increased flatulence, poor stool consistency, or gut inflammation, gut discomfort, microbial dysbiosis and/or indigestion, faecal malodour and for improving nutrient absorption, intestinal fermentation, and/or immunity in an animal, as defined by the claims.

### BRIEF DESCRIPTION OF THE FIGURES

Fi. 1 shows the anti-adhesion effect on Salmonella enterica caused by combinations of SDP and FOS at different content and ratios after 4 h incubation with ingredients.

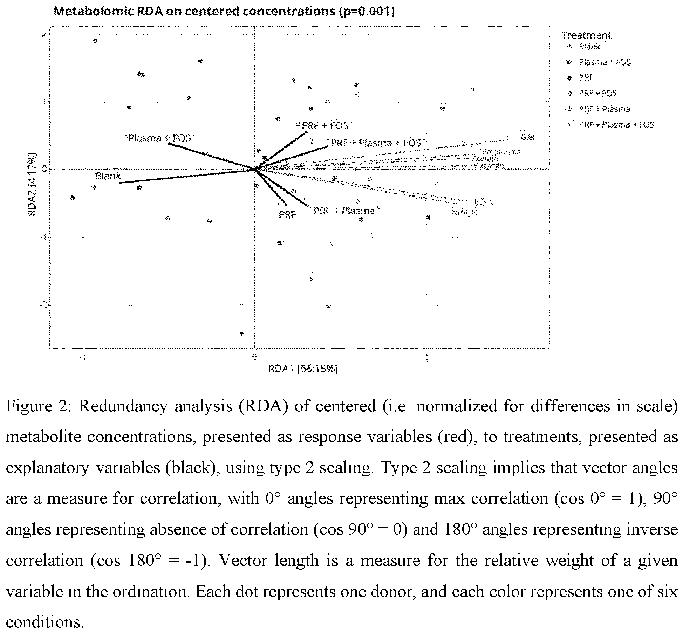

Fig. 2 shows a redundancy analysis (RDA) of centered (i.e. normalized for differences in scale) metabolite concentrations, presented as response variables (red), to treatments, presented as explanatory variables (black), using type 2 scaling. Type 2 scaling implies that vector angles are a measure for correlation, with 0° angles representing max correlation (cos 0° = 1), 90° angles representing absence of correlation (cos 90° = 0) and 180° angles representing inverse correlation (cos 180° = -1). Vector length is a measure for the relative weight of a given variable in the ordination. Each dot represents one donor, and each color represents one of six conditions.

### DETAILED DESCRIPTION OF THE INVENTION

The present invention provides an animal food composition comprising spray-dried plasma (SDP) and fructo-oligosaccharides (FOS) in a ratio of from 10 : 2 to 10 : 4.

During the extensive studies leading to the present invention the inventors examined the effect of various combinations of different amounts of SDP with different prebiotics in different amounts each on gastric and intestinal digestion, protein digestibility, nutrient absorption, gut microbiome modulation, total and individual gas production and intestinal health and surprisingly found that a combination of SDP and FOS in a ratio of between 10 : 2 to 10 : 4 yields the best results, in particular as regards a reduction of the total and malodour creating faecal compounds associated with the gut microbiome and increasing intestinal health. Without wishing to be bound by any theory it is presently contemplated that these ingredients interact synergistically in these amounts.

As the SDP to be included in the present animal foods any SDP nowadays commercially available may be used.

The fructo-oligosaccharides (FOS) to be used in the present invention may be obtained from any natural source, such as via extraction from fruits and vegetables, e.g. bananas, onions, chicory, asparagus or Jerusalem artichoke and/or obtained via degradation of inulin or produced by transfructosylation action of a β-fructosidase of Aspergillus niger on sucrose. In the context of the present invention the term fructo-oligosaccharides comprises any compound designated by the generic formula Glu-Fruₙ and/or Fruₙ with n being an integer from 1 - 7, and any mixture of these compounds in any individual ratio.

The ratio of SDP : FOS is in a range of from 10 : 2 - 10 : 4, with a ratio of 10 : 2,5 - 10 : 3.5 of 10 : 2,8 - 10 : 3.2 being preferred. A ratio of 10 : 3 is most preferred.

Depending on the nature of the animal food composition the SDP may be included therein in an amount of from 0.5 - 90 , preferably of from 0.5 - 80 %, more preferred 0.5 - 70 % or 60 or 50 or 40 or 30 or 20 or 10 %. Preferred ranges are 0.5 - 5 % or 0.5 - 3 % or 0.5 to 2 or 0.5 to 1 % all based on the total weight of the animal food composition, either dry or wet weight.

Likewise the amount of FOS in an animal food composition may range of from 0.1 % and up to 45 %, preferably 0.1 % - 40, 30, 20, 10, 5, 3, 2, 1, 0.5 % or 0.15 % - 40, 30, 20, 10, 5, 3, 2, 1, 0.5 %. A preferred range is also 0.15 % - 0.3 % all based on the total weight of the animal food composition, either dry or wet weight.

In the context of the present invention the term animal food composition is to be understood as comprising a ready to use animal food or also as an additive to be included in a conventional animal food, a supplement, a topper, or treat products. The animal food composition may be embodied as a dry food, a wet food, granules, powder, pellets, chews, paste, liquid depending on the desired mode of administration of the food.

The animal, to which the food may be administered is a pet or livestock, such as dogs, cats or rabbits, swine, cattle, goat, sheep, chicken, geese, ducks, turkeys. According to a preferred embodiment the present composition is a pet food, in particular a dog or cat food.

The present compositions may be used in maintaining the gut health, treating gut disorders and maintaining and increasing the animal's immune status. In particular, the present compositions may be used for treating and/or preventing a gut disorder, such as increased flatulence or gut inflammation, gut discomfort, microbial dysbiosis and/or indigestion and for improving microbiota balance, nutrient absorption, intestinal fermentation, and/or immunity in an animal.

In particular the compositions are useful for preventing or treating immunological diseases and disorders due to inflammatory factors secretion affecting an animal subject.

For animals, inclusive pets, the gastrointestinal mucosa constitutes a critical barrier where millions of microbes and environmental antigens come in close contact with the immune system. The GI mucosa, essentially comprised by the outer mucus layer with the commensal gut microbiota, antimicrobial proteins and secretory immunoglobulin A molecules, a central single cell layer with specialised epithelial cells, and the inner lamina propria where innate and adaptive immune cells reside, has the complex task to act as a semipermeable barrier that allows absorption of nutrients and immune sensing, while limiting the transport of potentially harmful compounds and microorganisms.

This function can be compromised by changing the microbiota due to nutritional variations and by harmful compounds produced and secreted by micro-organisms, such as e.g. ammonia, indoles, phenols, and sulphur-containing compounds.

As found in the present invention, compositions comprising the ratio of SDP and FOS in the given ratios strengthen the intestinal barrier's integrity, in particular the central epithelial layer by obviously supporting maintenance of tight junctions directly by food derivatives and by microbial metabolites, as documented by a reduced permeability of a epithelial monolayer determined via TEER.

Since microbiota is obviously shifted to utilise FOS rather than protein at the same time, the present compositions assist in maintaining and/or restoring a healthy gut condition. Without wishing to be bound by any theory this observation could in part be based in a reduced formation of potentially harmful and/or malodourous substances in the intestine and at the same time preventing or reducing permeation thereof through the inner epithelial layer. As such, the activity of the immune cells in the lamina propria is not substantially impaired and these cells may continue undisturbed with their inherent task.

According to a preferred embodiment the gut disorder is selected from flatulence, increased flatulence or inflammatory conditions, such as e.g. created by manifestation of harmful micro-organisms.

Due to the bioactive substances contained in SDP and at the same time reducing any adverse effects potentially brought about by the food the immune status of the animal may be improved.

### Examples

### Example 1: Effect on intestinal health

In order to examine the influence of compositions as detailed herein on the health status of the intestinal barrier, trans epithelial electrical resistance (TEER) measurements have been carried out. Such an assay is an acknowledged model for reflecting the status of a cellular barrier, as present in the intestine, wherein the resistance over an artificial barrier is measured.

For preparing a cell monolayer, Caco-2 cells were trypsinised at 70-80% confluence and seeded in culturing DMEM medium into 12-well Transwell^{®} plates at a density 6 × 10⁴ cells per insert. Cells were incubated at 37 °C for 21 days to allow differentiation, with media changed every 2-3 days in both, the apical and basolateral compartments formed by the monolayer of Caco-2 cells. TEER was measured once per week using a Millicell^{®}-ERS voltammeter (EMD Millipore, USA). On day 21 the formed monolayers were rinsed twice with HBSS and 1.5 ml HBSS was added to the basolateral compartment.

The apical compartment was filled with samples prepared as follows.

SDP, standard dog food, dog food containing 2% of SDP, dog food containing 0.6% FOS, and dog food containing 2 % of SPD:FOS in a ratio of 10:3 was subjected to a simulated canine digestion using porcine pepsin, porcine pancreatin, bovine bile salts and mineral salts to represent digestive conditions of medium size dog.

The gastric phase was carried at a pH of 2 and 39 °C for about 2 hrs and intestinal phase at pH 6.8 and 39 °C for a time period of about 4 hrs. To stop the digestion process and to inactivate the enzymes, the inhibitors orlistat and Pefabloc were added at the end of the intestinal phase in final concentrations of 0.1 mM and 1.1 mM respectively, after which all samples were immediately snap-frozen for storage at -80°C.

On the day of the cell culture experiment the samples obtained as above were thawed and diluted 5-fold with HBSS, filter-sterilised and added to the apical side of monolayer at 0.5 ml volume. As negative controls, HBSS alone and blank digesta formulation (enzymes without food substrate), also 5-fold diluted with HBSS, were added to the apical side.

The experimental set up as detailed above was incubated for 4 hrs at 37 °C, followed by collecting apical and basolateral supernatants for storage at -80 °C. TEER measurements were performed before/after washing and after 4 h treatment.

The results are summarized in table 2 below.

**Table 2**

| Sample | HBSS | Blank digesta | SDP | Dog food | Dog food + 2 % SDP | Dog food + 0.6 % FOS | Dog food + 2 % SDP:FOS (10:3) |
|---|---|---|---|---|---|---|---|
| TEER | 100 % | 98 % | 110 % | 92 % | 103 % | 107 % | 119 % |

As evident from the above, addition of SDP and FOS to dog food in a ratio of 10:3 yielded the best results, in maintaining the tight junctions between the cells.

### Example 2: Effect on immunity

In order to examine the influence of compositions as detailed herein on prevention of infection, reduction in adhesion of salmonella to the modelled intestinal barrier was measured. Such an assay is an acknowledged model for testing compounds, which can affect bacteria - host interaction and reduce probability of getting infected by reducing surface attachment sites.

For preparing a cell monolayer, HT29-MTX cells were trypsinised at 90% confluence and seeded in culturing DMEM medium (10%FBS, 1% NEAA) into Corning CellBind 12-well plates at a density 5 × 10⁴ cells per well. Cells were incubated at 37 °C and 5% CO₂ for 10 days to allow differentiation, with media changed every 2-3 days. Media was changed to 2% FBS DMEM (1% NEAA) 24 h prior to experiment.

Pre-culture of *Salmonella enterica* was prepared a day before experiment by defrosting cryovial, mixing and transferring 10 µl by loop to 10 mL BHI. After gentle vortexing it was incubated at 37°C (aerobic conditions) for 8 h. Overnight culture of *Salmonella enterica* was prepared by transferring 100 µl of pre-culture to a new sterile glass tube containing 10 mL BHI.

Samples of SDP, standard dog food, dog food containing 2% of SDP, dog food containing 0.6% FOS, and dog food containing 2 % of SPD:FOS in a ratio of 10:3 was subjected to a simulated canine digestion as described above. Samples were prepared for cell exposure by diluting 1:5 in 2% FBS DMEM (1% NEAA) media and filter-sterilising. Cells were washed by PBS and incubated with prepared samples for 4h.

Overnight culture (0.25 mL) was mixed with 19.75 mL DMEM (2% FBS) to create solution for use in experiments. Cells were washed 3 times with PBS and incubated with 0.52 mL of bacterial solution for 45 minutes in aerobic conditions. Following incubation period, non-adherent bacteria were removed by washings 3 times with PBS. Cells were lysed by 0.5 mL of 0.1% Triton-X at room temperature for 30 minutes. Time 0 and post-incubation cell lysates were plated in serial dilutions (-4,-5,-6) on to BHI agar plates and incubate at 37°C (aerobic conditions) overnight. Colonies were counted using a colony counter and the number of colony forming units per mL calculated according to the formula: $CFU / mL = \frac{average no . of colonies counted}{dilution \times plated volume \left(0.01mL\right)}$

The number of adherent bacteria was calculated as a percentage of the initial inoculum (T0): $Percentage of adherent bacteria = \frac{CFU / mL cell lysate}{CFU / mL initial inoculim} \times 100$

Results were plotted as fold adhesion increase versus the non-supplemented control: $Fold adhesion increase = \frac{% adherent bacteria test sample}{% adherent bacteria NS control}$

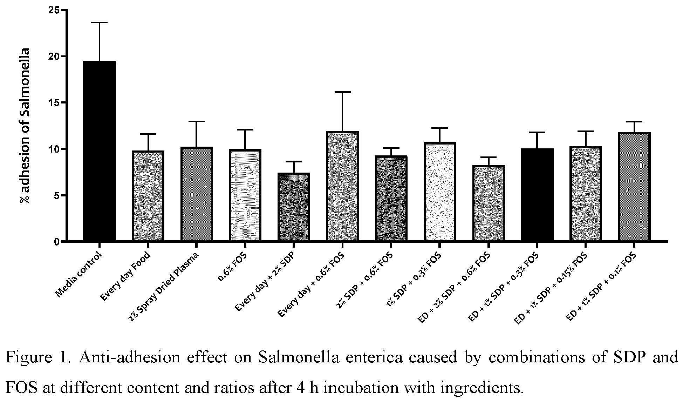

The results are summarised in Figure 1.

All ingredients reduced adhesion of *Salmonella,* most pronounced effect observed with every day food + 2% spray dried plasma (with or without 0.6% FOS).

### Example 3: Shift of faecal fermentation from proteolytic to saccharolytic

To assess shift of faecal fermentation from proteolytic to saccharolytic in the presence of FOS a short-term incubation of test compound with fecal inocula of nine dog donors as microbial sources was performed under conditions representative for the large intestine.

The test products were added to the reaction vessels in the presence or absence of the dog food (PRF) in the ratio 10:3 SDP:FOS or in corresponding concentrations individually. For conditions with PRF, pre-digested PRF was added, while for the canine conditions not requiring PRF addition, blank pre-digested medium was supplemented. Finally, 10% (v/v) of a cryopreserved fecal inoculum suspension, containing 20% (w/v) canine fecal material was added per reactor and incubated at 39°C under continuous shaking and anaerobic atmosphere.

The enrichment of proteolytic markers (NH4-N and bCFA) was comparable between PRF and PRF+plasma, implying that mostly the PRF fraction (more specifically the product's protein fraction) was responsible for this enrichment. 'PRF' and 'PRF + Plasma' had a similar impact on the fermentation, as did 'PRF + FOS' and 'PRF + Plasma + FOS'. FOS addition to 'PRF' or 'PRF + Plasma' further improved gut-health, as it resulted in a higher production of the saccharolytic markers (acetate, propionate and butyrate), and lower production of proteolytic markers. In contrast, 'Plasma + FOS' had the lowest impact on the various fermentation parameters due to the absence of PRF and high protein content correspondingly, making the baseline fermentation different and resulting in less pronounced shift. 'PRF + Plasma + FOS' resulted in the highest production of gas, and of the SCFA acetate, propionate, and butyrate. Based on these observations, there is clear merit in combining PRF, plasma and FOS, making this product combination the superior product, optimising faecal fermentation and reducing stool odour associated with proteolytic fermentation.

### Example 4: Canine faecal malodour

25 dogs (beagles) of an age range of between 2 - 6 years were split into five groups of 5 dogs each, which were separately held and fed with conventional dog food for a period of 3 days and subsequently with the same dog food supplemented with 2 % SDP : FOS (based on the weight of the dog food) in different ratios also for 3 days each. Feeding was effect once a day at 7:00 AM.

The living area of the dogs was cleared of any faeces at the end of a day (5 PM). The faeces samples on day 3 after initiating a feed course were collected, transferred into plastic bags and stored at 4 °C until examination.

Stool odour determinations were assessed using a 1-to-5 scoring scale, with a classification into five (very slightly odorous (very acceptable); +), slightly odorous (acceptable): ++), mildly odorous (slightly acceptable); +++), odorous (slightly bearable); ++++), and very odorous (unbearable); +++++)). The results of these measurements are summarized in table 1 below.

**Table 1**

| Groups | 1 | 2 | 3 | 4 | 5 |
|---|---|---|---|---|---|
| Dog food | ++++ | +++++ | ++++ | ++++ | ++++ |
| + SDP:FOS | 10:1 | 10:2 | 10:3 | 10:4 | 10:5 |
| | +++++ | ++ | + | ++ | ++++ |
| + SDP:FOS | 20:3 | 15:3 | 10:3 | 5:3 | 1:3 |
| | | ++ | + | ++++ | |

As is evident from the above, a clear drop in malodour could be sensed, when combining SDP and FOS in a range as from 10:2 - 10:4.

## Claims

1. An animal food composition comprising spray-dried plasma (SDP) and fructo-oligosaccharides (FOS) in a ratio of from 10 : 2 to 10 : 4.

2. The animal food composition of claim 1, wherein the ratio of SDP : FOS is in a range of from 10 : 2.5 - 10 : 3.5, preferably 10 : 3.

3. The animal food composition of any of claims 1 - 2, wherein the SDP is contained in the animal food in an amount of from 0.5 - 90 wt.-% based on the total weight of the composition.

4. The animal food composition according to any of the preceding claims, wherein the FOS is contained in the animal food in an amount of from 0.1 - 45 wt.-% based on the total weight of the composition.

5. The animal food composition according to any of the preceding claims, which is formulated as a ready-to use food, supplement, additive or topper.

6. The animal food composition according to any of the preceding claims, wherein the animal is a pet or livestock, preferably a cat or dog.

7. An animal food composition according to any of the preceding claims for use in maintaining the gut health, treating gut disorders, gut inflammation, gut discomfort, and/or maintaining or increasing immunity in an animal.

8. The animal food composition for use according to claim 7, wherein the gut disorder is selected from increased flatulence or gut inflammation.

9. Non-therapeutic use of an animal food composition of any of claims 1-6 for improving nutrient absorption, intestinal fermentation in an animal and reducing faecal odour; and/or improving food digestibility and microbiota balance.

10. The non-therapeutic use according to claim 9, wherein the animal is a pet or a livestock, preferably a cat or a dog.

## Patentansprüche

1. Tierfutterzusammensetzung, welche sprühgetrocknetes Plasma (SDP) und Fructo-Oligosaccharide (FOS) in einem Verhältnis von 10 : 2 bis 10 : 4 umfasst.

2. Tierfutterzusammensetzung nach Anspruch 1, worin das Verhältnis von SDP : FOS im Bereich von 10 : 2,5 bis 10 : 3,5, vorzugsweise 10 : 3, liegt.

3. Tierfutterzusammensetzung nach einem der Ansprüche 1-2, worin das SDP in dem Tierfutter in einer Menge von 0,5 bis 90 Gew.-%, bezogen auf das Gesamtgewicht der Zusammensetzung, enthalten ist.

4. Tierfutterzusammensetzung nach einem der vorstehenden Ansprüche, worin das FOS in dem Tierfutter in einer Menge von 0,1 bis 45 Gew.-%, bezogen auf das Gesamtgewicht der Zusammensetzung, enthalten ist.

5. Tierfutterzusammensetzung nach einem der vorstehenden Ansprüche, welche als gebrauchsfertiges Futter, Ergänzungsfuttermittel, Zusatzstoff oder Topping formuliert ist.

6. Tierfutterzusammensetzung nach einem der vorstehenden Ansprüche, worin es sich bei dem Tier um ein Haustier oder ein Nutztier, vorzugsweise um eine Katze oder einen Hund, handelt.

7. Tierfutterzusammensetzung gemäß einem der vorstehenden Ansprüche zur Verwendung bei der Erhaltung der Darmgesundheit, zur Behandlung von Darmerkrankungen, Darmentzündungen, Darmbeschwerden und/oder zur Erhaltung oder Stärkung der Immunität bei einem Tier.

8. Tierfutterzusammensetzung zur Verwendung gemäß Anspruch 7, worin die Darmerkrankung ausgewählt ist unter vermehrten Blähungen oder einer Darmentzündung.

9. Nichttherapeutische Verwendung einer Tierfutterzusammensetzung nach einem der Ansprüche 1-6,
zur Verbesserung der Nährstoffaufnahme und Fermentierung im Darm bei einem Tier sowie zur Verringerung des Kotgeruchs; und/oder
zur Verbesserung der Futterverdaulichkeit und des Gleichgewichts der Mikrobiota.

10. Nichttherapeutische Verwendung nach Anspruch 9, worin das Tier ein Haustier oder ein Nutztier ist, vorzugsweise eine Katze oder ein Hund.

## Revendications

1. Composition alimentaire pour animaux comprenant du plasma séché par atomisation (SDP) et des fructo-oligosaccharides (FOS) dans un rapport compris entre 10 : 2 et 10 : 4.

2. Composition alimentaire pour animaux selon la revendication 1, dans laquelle le rapport SDP : FOS se situe dans une plage comprise entre 10 : 2,5 et 10 : 3,5, de préférence 10 : 3.

3. Composition alimentaire pour animaux selon l'une quelconque des revendications 1-2, dans laquelle le SDP est présent dans l'aliment pour animaux en une quantité comprise entre 0,5 et 90% en poids par rapport au poids total de la composition.

4. Composition alimentaire pour animaux selon l'une quelconque des revendications précédentes, dans laquelle le FOS est présent dans l'aliment pour animaux en une quantité comprise entre 0,1 et 45% en poids par rapport au poids total de la composition.

5. Composition alimentaire pour animaux selon l'une quelconque des revendications précédentes, qui est formulée sous forme d'aliment prêt à l'emploi, de complément alimentaire, d'additif ou de garniture.

6. Composition alimentaire pour animaux selon l'une quelconque des revendications précédentes, dans laquelle l'animal est un animal de compagnie ou un animal d'élevage, de préférence un chat ou un chien.

7. Composition alimentaire pour animaux selon l'une quelconque des revendications précédentes, destinée à être utilisée pour maintenir la santé intestinale, traiter les troubles intestinaux, l'inflammation intestinale, les tensions intestinaux et/ou maintenir ou renforcer l'immunité chez un animal.

8. Composition alimentaire pour animaux destinée à être utilisée selon la revendication 7, dans laquelle le trouble intestinal est choisi parmi une augmentation des flatulences ou une inflammation intestinale.

9. Utilisation non thérapeutique d'une composition alimentaire pour animaux selon l'une quelconque des revendications 1-6, destinée
à améliorer l'absorption des nutriments et la fermentation intestinale chez un animal, ainsi qu'à réduire l'odeur des selles; et/ou
à améliorer la digestibilité des aliments et l'équilibre du microbiote.

10. Utilisation non thérapeutique selon la revendication 9, dans laquelle l'animal est un animal de compagnie ou un animal d'élevage, de préférence un chat ou un chien.
